# FASCICULE DE BREVET EUROPEEN

(11) **EP 2 864 044 B1**
(45) Date de publication et mention de la délivrance du brevet: **25.12.2019**
(21) Numéro de dépôt: 13744613.4
(22) Date de dépôt: 24.06.2013
(51) Int. Cl.: B01J 27/224, B01J 21/06, B01J 23/75, B01J 23/745, B01J 35/04, B01J 35/00, C10G 2/00, B01J 37/02

(54) **Supports de catalyseur à base de carbure de silicium recouvert de TiO2 pour la synthèse de Fischer-Tropsch**
KATALYSATORTRÄGER AUS MIT TITANDIOXID BESCHICHTETEM SILIZIUMKARBID UND SEINE VERWENDUNG IN FISCHER-TROPSCH SYNTHESE
CATALYST CARRIER COMPRISING SILICON CARBIDE COATED WITH TITANIUM DIOXIDE AND ITS USE IN FISCHER-TROPSCH SYNTHESIS

(30) Priorité: 26.06.2012 FR 1256028; 31.07.2012 FR 1257446
(43) Date de publication de la demande: 29.04.2015
(73) Titulaire: Sicat Catalysts, Inc., Wilmington DE 19801 (US); CENTRE NATIONAL DE LA RECHERCHE SCIENTIFIQUE -CNRS-, 75016 Paris (FR); Université de Strasbourg, 67000 Strasbourg (FR)
(72) Inventeur: LIU, Yuefeng, F-67000 Strasbourg (FR); PHAM-HUU, Cuong, F-67100 Strasbourg (FR); NGUYEN, Patrick, F-67000 Strasbourg (FR); PHAM, Charlotte, F-67000 Strasbourg (FR)
(74) Mandataire: Schmidt, Martin Peter
(86) Numéro de dépôt international: PCT/FR2013/051465
(87) Numéro de publication internationale: WO 2014/001697

(56) Documents cités:
- WO-A1-2010/029235
- WO-A1-2012/038621
- WO-A2-2009/098393
- FR-A1- 2 887 545
- US-A1- 2006 110 308
- KOUAMÉ: "Preliminary study of the use of [beta]-SiC foam as a photocatalytic support for water treatment", CATALYSIS TODAY, 1 janvier 2010 (2010-01-01), XP055012083, ISSN: 0920-5861
- PATRICK NGUYEN ET AL: "ChemInform Abstract: Innovative Porous SiC-Based Materials: From Nanoscopic Understandings to Tunable Carriers Serving Catalytic Needs", CHEMINFORM, vol. 42, no. 18, 3 mai 2011 (2011-05-03), pages no-no, XP055012066, ISSN: 0931-7597, DOI: 10.1002/chin.201118188
- RODRIGUEZ P ET AL: "Deposition and characterisation of TiO"2 coatings on various supports for structured (photo)catalytic reactors", APPLIED CATALYSIS A: GENERAL, ELSEVIER, AMSTERDAM, NL, vol. 360, no. 2, 1 June 2009 (2009-06-01), pages 154-162, XP026089926, ISSN: 0926-860X, DOI: 10.1016/J.APCATA.2009.03.013 [retrieved on 2009-03-20]

## Description

### Domaine technique de l'invention

L'invention concerne la catalyse hétérogène, et plus particulièrement pour la réaction de Fischer-Tropsch, d'un catalyseur obtenu par le dépôt d'une phase active sur un support de catalyseur, ladite phase active étant du cobalt métallique, du fer métallique ou un mélange des deux, finement divisés, ledit support de catalyseur étant un support de catalyseur à base SiC. Ce support de catalyseur appartient aux supports poreux à base de carbure de silicium (SiC), en particulier à base de β-SiC, modifiés par un dépôt de surface de TiO₂.

### Etat de la technique

La réaction de Fischer-Tropsch convertit un mélange de CO et d'hydrogène en hydrocarbures. Il existe deux types de catalyseurs pour la réaction Fischer-Tropsch : les catalyseurs à base de fer, qui travaillent de manière optimale à une température de l'ordre de 350°C (dits « catalyseur FT de haute température »), et les catalyseurs à base de cobalt, qui travaillent à plus basse température, en général inférieure à 250°C. Ils sont majoritairement composés d'une phase active et d'un support oxyde. Un résumé de l'état de la technique sur les catalyseurs pour la réaction FT à base de cobalt est donné dans l'article « *On the selectivity of cobalt-based Fischer-Tropsch catalysts : Evidence for a common precursor for methane and long-chain hydrocarbons »* par S. Lôgdberg et al., paru en 2010 dans J. Catalysis (doi :10.1016/jcat.2010.06.007). Les supports oxydes sont majoritairement constitués d'alumine, de silice et optionnellement de dioxyde de titane. Les supports oxydes présentent d'excellentes propriétés permettant de confectionner des catalyseurs actifs pour la réaction FT, mais ils souffrent aussi de désavantages tels que leur très faible conductivité thermique, une faible résistance hydrothermale, la présence de sites acides en surface (alumine), une faible résistance mécanique en particulier pour des extrudés (silice et dioxyde de titane) et une faible résistance à l'attrition pour des microbilles utilisées en lit bouillonnant (en particulier pour la silice et le TiO₂).

Afin d'accroitre la stabilité mécanique et hydrothermale des supports oxydes, on a cherché à modifier ces supports. On peut citer l'alumine promue à l'oxyde de lanthane La₂O₃ (brevets US 5,537,945 et US 6,255,358 (Energy International Corp.), US 7,163,963 (Conoco Phillips Co.)), au Si (demande de brevet US 2005/0124490 (Chevron Texaco Corp.), brevet US 7,365,040 (Sasol Technology)) et au Ti ou Zr (brevet US 6,975,7209 (Sasol Technology)). Des supports en spinelle ont été également proposés (demandes de brevet WO 2006/067285 (Institut Français du Pétrole) et US 2007/0161714), ainsi que des supports en silice amorphe (demande de brevet US 2010/0311570) pour accroître la résistance à l'attrition. L'alumine utilisée est en général majoritairement de l'alumine-y, mais on a également utilisé des supports en alumine comprenant une majorité d'alumine-a (brevet US 7,351,679 (Statoil ASA)).

Il a été reporté dans la littérature que les supports en TiO₂ permettent de fabriquer des catalyseurs à base de cobalt extrêmement sélectifs (C₅₊) pour la FT (voir les publications parues dans la revue Catalysis Today, vol. 100 (2005), p. 343-347 et dans la revue Journal of Catalysis, vol. 236 (2005), p. 139, ainsi que dans Applied Catalysis A: General, vol. 210 (2001) p. 137-150). Cependant, les auteurs mentionnent que leur résistance mécanique est trop faible.

Le TiO₂ est utilisé généralement pour augmenter les interactions avec les particules de la phase active ; cela est connu par exemple de WO 2009/098393. Le TiO₂ déposé sur un support de SiC a également été exploré comme photocatalyseur, voir la publication de Koaumé et al ; parue dans Catalysis Today 161 (2011) p. 3-7. En tant que tel le SiC est connu comme support de catalyseur, voir US 2006/0110308. Il a été reporté dans la littérature qu'un support de silice recouvert d'une couche de TiO₂ permettait d'accroitre l'activité et la sélectivité d'un support de silice. Les résultats obtenus sur ce type de support à base de silice promu avec du TiO₂ (voir la publication « Influence of Support Preparation Methods on Structure and Catalytic Activity of Co/TiO2-SiO2 for Fischer-Tropsch Synthesis » de S. Mu et al., parue en 2009 dans le revue Catal. Lett. 133, p. 341-345, et la publication de S. Hinchiranan et al. parue en 2008 dans la revue Fuel Proc. Technol. 89, p. 455-459) font état d'une amélioration sensible de l'activité catalytique en synthèse FT. L'introduction du TiO₂ dans le support modifie également la sélectivité en hydrocarbures liquides. Néanmoins, en fonction du mode de dépôt et de la configuration du test FT, lit fixe ou lit bullant, l'influence est différente, e.g. une amélioration dans le cas d'un test FT en mode lit fixe et une légère chute dans le cas d'un test en mode lit agité. Dans tous les cas, l'amélioration de l'activité catalytique en FT est attribuée à une meilleure dispersion des particules de cobalt avec une taille plus faible en présence du TiO₂.

Enfin, dans des réacteurs tubulaires lits fixes, l'utilisation des catalyseurs supportés sur β-SiC permet de tempérer les variations thermiques dans le lit catalytique grâce à la forte conductivité thermique du matériau β-SiC. L'ensemble de ces avantages permettent d'opérer la synthèse FT dans des conditions plus sévères afin d'améliorer la productivité du procédé.

Un support de catalyseur appartient aux supports poreux à base de carbure de silicium (SiC), en particulier à base de β-SiC, modifiés par un dépôt de surface de TiO₂ est décrit dans la revue Applied Catalysis A: General vol. 360 (2009), p. 154-162 ainsi que dans Applied Catalysis A: General vol. 391 (2011), p.443-454.

La présente invention a pour but de mettre œuvre une réaction de Fischer-Tropsch avec un catalyseur présentant une meilleur stabilité et un meilleur rendement ainsi qu'une meilleure sélectivité.

La réaction de Fischer-Tropsch est un procédé de conversion de monoxyde de carbone (CO) et d'hydrogène (H2) en hydrocarbures.

### Objet de l'invention

Selon l'invention, le problème est résolu en déposant la phase active sur une couche d'oxyde de titane (TiO₂) très finement divisée, constituée de nanoparticules, qui recouvre au moins en partie la surface macroporeuse et méso-poreuse du support poreux en SiC. Le carbure de silicium est de préférence du β-SiC présentant au moins une macroporosité et une méso-porosité. Il peut s'agir par exemple de granulés, de billes ou d'une mousse de β-SiC. Le TiO₂ est de préférence l'anatase.

Les inventeurs ont observé que lorsque le cobalt est déposé sur un support à base de carbure de silicium (et en particulier sur une mousse alvéolaire en β-SiC) recouvert de TiO₂, cela permet d'augmenter d'une manière significative l'activité en synthèse FT, tout en maintenant une sélectivité en hydrocarbures liquides relativement élevée supérieure à 90%. La méthode de dépôt de la couche superficielle de TiO₂ est facile à mettre en œuvre et ce pour tous types et formes de support.

Ce support mixte possède les propriétés intéressantes provenant à la fois du polytype beta du carbure de silicium (β-SiC) et du dioxyde de titane en particulier sous sa forme anatase. Parmi ces propriétés, on peut citer pour le β-SiC son excellente conductivité thermique, une porosité bimodale méso et macroporeuse, une excellente résistance chimique et hydrothermale ainsi qu'une forte résistance mécanique. Le dioxyde de titane sous sa forme anatase permet la dispersion fine de particules de phase active.

La méthode de synthèse dudit support permet de contrôler à volonté la morphologie et la taille des supports en fonction de la configuration des réacteurs utilisés. Les catalyseurs préparés à partir de ces supports recouverts de TiO₂ présentent une activité élevée en SFT en comparaison de celle mesurée sur un catalyseur homologue déposé sur β-SiC sans couche de TiO₂.De plus, les performances catalytiques de ces catalyseurs supportés sur β-SiC recouvert de TiO₂ sont très stables dans le temps.

Selon un mode de préparation dudit support le dépôt de TiO₂ est réalisé par un procédé de type sol-gel faisant intervenir une phase liquide d'un précurseur de TiO₂.Cela donne de meilleurs résultats comparé à un dépôt de cristaux de TiO₂, par exemple à partir d'une dispersion de poudre de TiO₂ (procédé connu conduisant à un dépôt appelé « wash-coat »). Plus particulièrement, ce procédé implique les étapes suivantes :
a) on approvisionne un support en β-SiC de haute porosité, de préférence sous la forme d'extrudés, granules, billes, microbilles, ou de mousse alvéolaire,et dont la surface spécifique est d'au moins 15 m²/g, et de préférence d'au moins 20 m²/g,
(b) on prépare une solution d'au moins un précurseur de TiO₂,
(c) on imprègne ledit support par ladite solution,
(d) on sèche ledit support imprégné,
(e) on calcine ledit support imprégné pour transformer ledit précurseur de TîO₂ en TiO₂, à une température comprise entre 400 °C et 1000 °C, et de préférence entre 500 °C et 900 °C.

Ce procédé conduit à la formation de cristallites de TiO₂ sur la surface mésoporeuse et macroporeuse. Cela évite la formation d'une croûte sur la surface macroporeuse qui empêche l'accès des gaz réactionnels aux méso-pores et micropores. Cela est reflété par le fait que la distribution poreuse du support n'est pas modifiée de manière significative par le dépôt des cristallites de TiO₂ sur la surface mésoporeuse et macroporeuse du support.

La contribution microporeuse à la surface spécifique dudit support est avantageusement inférieure à 5 m²/g, et encore plus préférentiellement inférieure à 3,5 m²/g.

Ladite calcination (étape (e)) est effectuée de préférence à une température comprise entre 400°C et 1000°C, et de préférence entre 500°C et 900°C. La montée en température se fait avantageusement avec une pente comprise entre 1,5°C/min et 3,5°C/min, et de préférence entre 1,5°C/min et 2,5°C/min.

Le support de catalyseur susceptible d'être obtenu par le procédé décrit ci-dessus présente avantageusement une teneur massique (en %) en TiO₂ par rapport à celle du SiC (voir ci-dessous la section « Définitions ») comprise entre 3% et 30%, de préférence entre 5% et 20%, encore plus préférentiellement entre 8% et 16%, et de manière optimale entre 8% et 13%. Avantageusement, sa teneur en TiO₂ est inférieure à 0,02 g par m² de surface spécifique du support en SiC, et de préférence inférieure à 0,013 g par m² de surface spécifique du support en SiC, et encore plus préférentiellement inférieure à 0,009 g par m² de surface spécifique du support en SiC.

Sur le support de catalyseur susceptible d'être obtenu par le procédé décrit ci-dessus on dépose une phase active pour obtenir un catalyseur. Ladite phase active est du cobalt métallique, du fer métallique ou un mélange des deux, finement divisés. Avantageusement, le catalyseur comprend entre 5% et 40% massiques et de préférence entre 10% et 25% massiques (exprimés par rapport à la masse du support) de cobalt sur un support dont le pourcentage massique de TiO₂ / SiC est compris entre 5% et 20%, de préférence entre 8% et 16%, et encore plus préférentiellement entre 8% et 13%.

Ainsi, l'objet de l'invention est l'utilisation pour la réaction de Fischer-Tropsch d'un catalyseur obtenu par le dépôt d'une phase active sur un support de catalyseur, ladite phase active étant du cobalt métallique,du fer métallique ou un mélange des deux, finement divisés, ledit support de catalyseur étant un support de catalyseur à base SiC recouvert au moins partiellement de TiO₂ susceptible d'être obtenu par un procédé de préparation comprenant les étapes suivantes:
(a) on approvisionne un support en β-SiC de haute porosité, de préférence sous la forme d'extrudés, granules, billes, microbilles, ou de mousse alvéolaire, et dont la surface spécifique est d'au moins 15 m²/g, et de préférence d'au moins 20 m²/g,
(b) on prépare une solution d'au, moins un précurseur de TiO₂,
(c) on imprègne ledit support par ladite solution,
(d) on sèche ledit support imprégné,
(e) on calcine ledit support imprégné pour transformer ledit précurseur de TîO₂ en TiO₂, à une température comprise entre 400 °C et 1000 °C, et de préférence entre 500 °C et 900 °C.

Des modes de réalisation particuliers sont définis dans les revendications 2 à 5.

### Figures

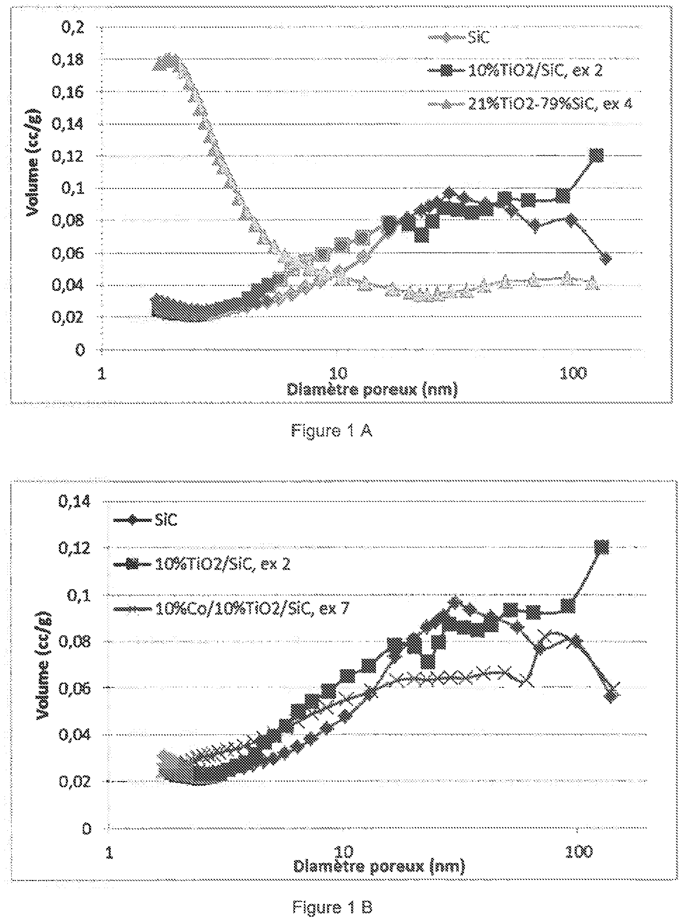

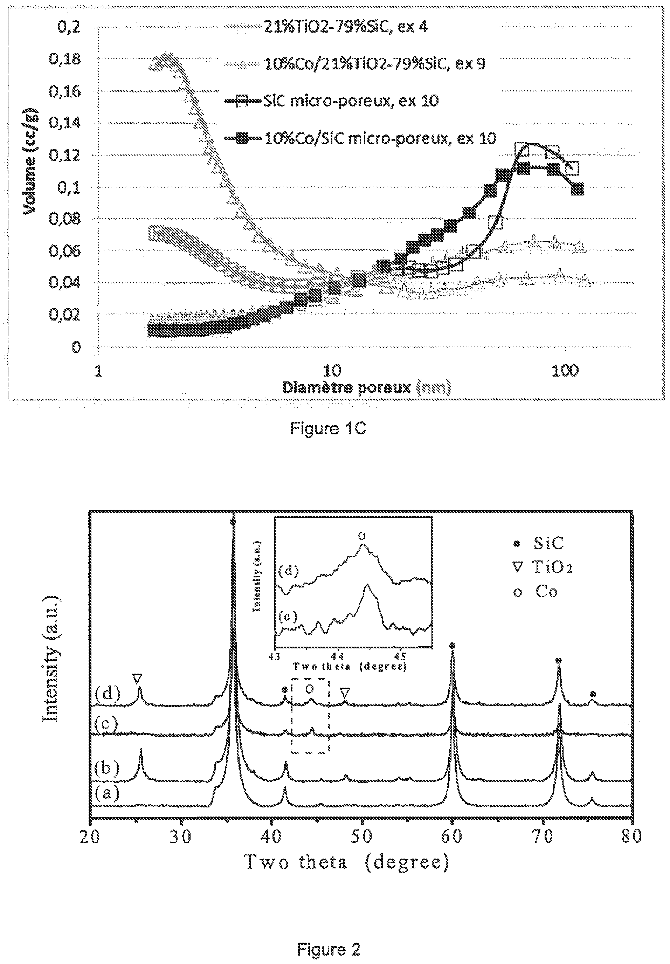

Les figures 1 à 11 illustrent des modes de réalisation de l'invention.
La figure 1 montre des courbes de distribution poreuse par sorptométrie à l'azote. La figure 1A montre ces courbes pour trois échantillons : β-SiC (losanges) ; 10% TiO₂ / β-SiC selon l'exemple 2 (carrés) ; 21% TiO₂ / 79% β-SiC / selon l'exemple 4 (triangles). La figure 1B montre, en abscisse dilatée, ces courbes pour β-SiC (losanges) et 10% TiO₂ / β-SiC selon l'exemple 2 (carrés), ces deux échantillons étant les mêmes que dans la figure 1A, et en plus la courbe correspondante pour 10% Co / 10% TiO₂ / β-SiC (croix) selon l'exemple 7. La figure 1C montre ces courbes pour quatre échantillons : SiC micro-poreux selon l'exemple 10 (carrés vides), SiC microporeux avec phase active de 10% de Co selon l'exemple 10 (carrés pleins), 21% TiO₂ - 79% β-SiC / selon l'exemple 4 (triangles vides), 10% Co / 21% TiO₂ - 79% SiC selon l'exemple 9.
La figure 2 montre les diagrammes de diffraction des rayons X des catalyseurs à base de cobalt supportés sur β-SiC et 10% TiO₂ / β-SiC après calcination sous air à 350 °C pendant 2 heures et une réduction sous H₂ à 300 °C. Pour comparaison les diagrammes du support seul, pur et recouvert de TiO₂, sont également présentés sur la même figure. Insert : Agrandissement sur le pic de diffraction du cobalt métallique montrant un élargissement de la largeur à mi-hauteur en présence du TiO₂. Echantillons : (a) β-SiC; (b) 10%TiO₂ / β-SiC (selon l'exemple 2) ; (c) 10% Co / β-SiC (selon l'exemple 5) ; (d) 10% Co / 10%TiO₂ / β-SiC (selon l'exemple 7).

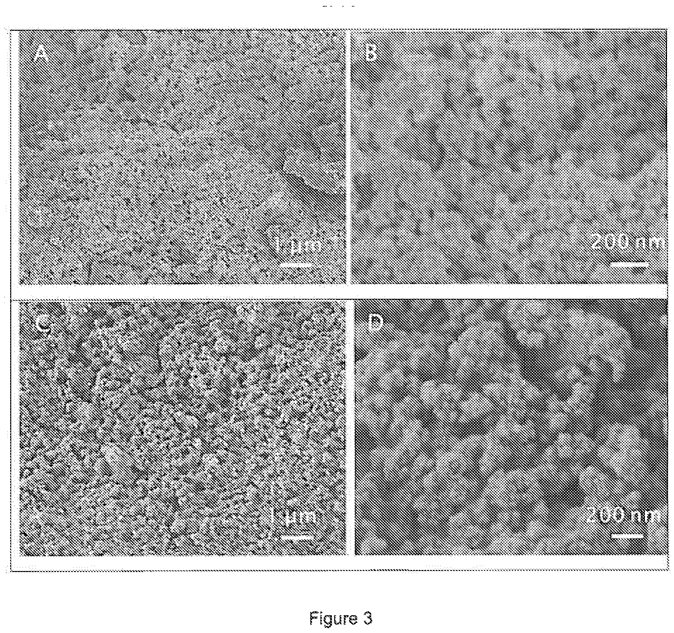

La figure 3 montre des micrographies obtenues par microscopie électronique à balayage (MEB) à différents agrandissements de deux échantillons: (A) et (B) : 10%Co / β-SiC (selon l'exemple 5). (C) et (D) : 10% Co / 10% TiO₂ / β-SiC (selon l'exemple 7). La longueur des barres est indiquée en bas à droite de chaque micrographie : 1 µm (A) et (C), 200 nm (B) et (D).

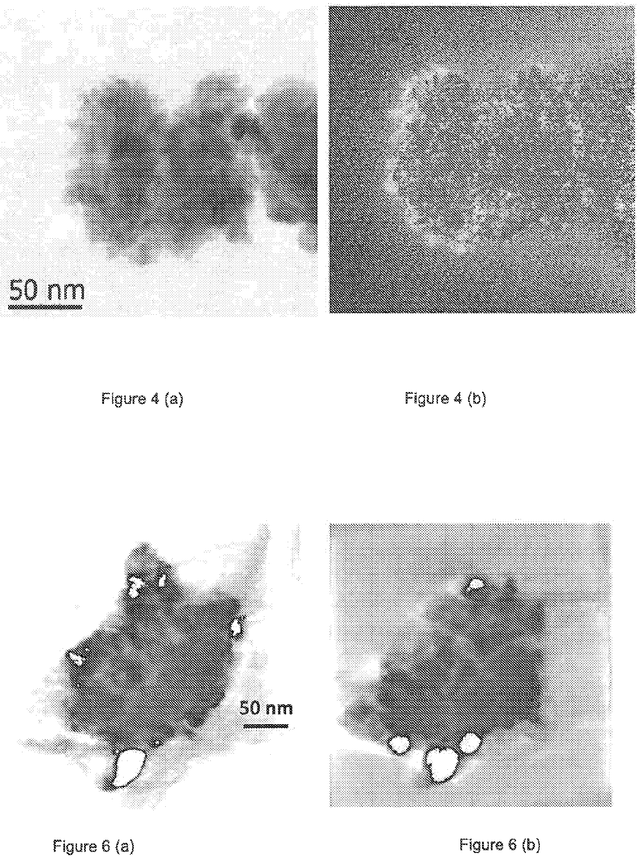

La figure 4 montre une micrographie obtenue par microscopie électronique à transmission (TEM) d'une zone du support 10% TiO₂ / β-SiC obtenu selon l'exemple 2. La figure 4(a) montre l'image TEM, la figure 4(b) la cartographie chimique obtenue en mode filtrage de l'énergie permettant d'identifier les zones contenant du Ti (en blanc) et celles contenant du Si (en foncé).. On observe que la couche de TiO₂ recouvre bien les particules de SiC. La longueur de la barre correspond à 50 nm.

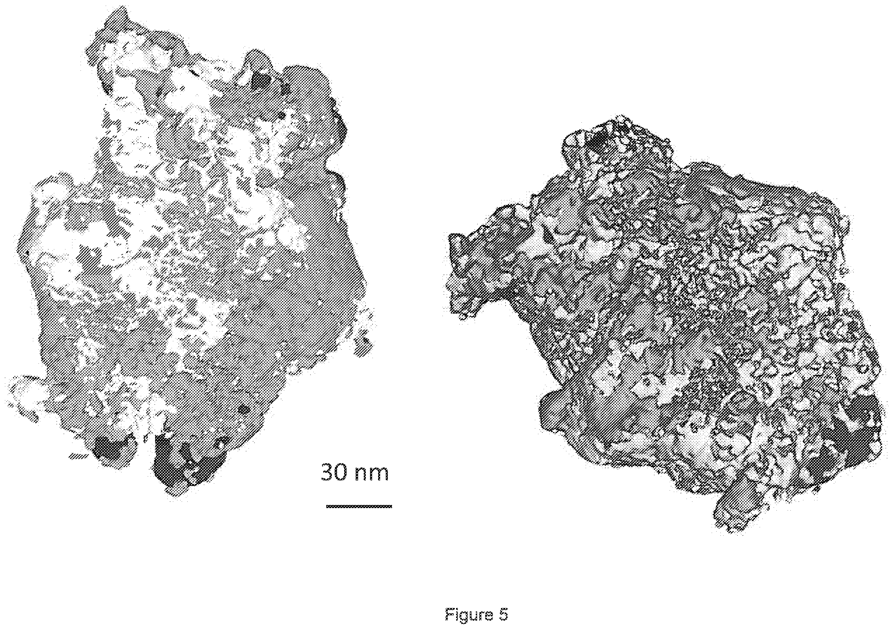

La figure 5 montre deux vues obtenues par microscopie électronique à transmission en mode filtrage de l'énergie (EFTEM) et en représentation tridimensionnelle d'une zone du catalyseur 10% Co / 21%TiO₂ - 79% β-SiC selon l'exemple 9 (comparatif) en mode cartographie chimique (les deux images correspondent au recto et verso de la même zone). On voit la répartition du TiO₂ (en blanc) et du SiC (en gris) qui se répartissent en surface du solide. On observe deux types de particules de cobalt (en noir): de grosses particules déposées sur les zones de SiC et quelques rares particules, nettement plus petites, déposées sur les zones de TiO₂.
La figure 6 montre deux autres micrographies obtenues par microscopie électronique à transmission en coupe transverse de deux particules (figures 6(a) et (b)) de catalyseur 10% Co / 21% TiO₂ - 79% β-SiC selon l'exemple 9 (comparatif). Les images ont été prises en mode spectroscopie de la perte d'énergie des électrons (EELS). Couleurs : blanc = Co ; noir = Ti ; gris = Si.

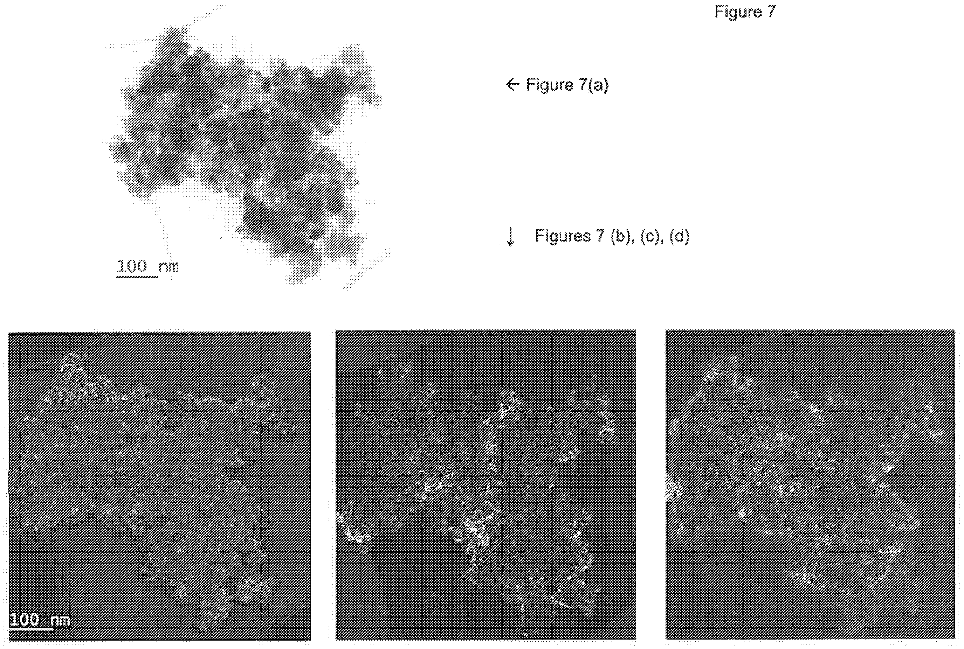

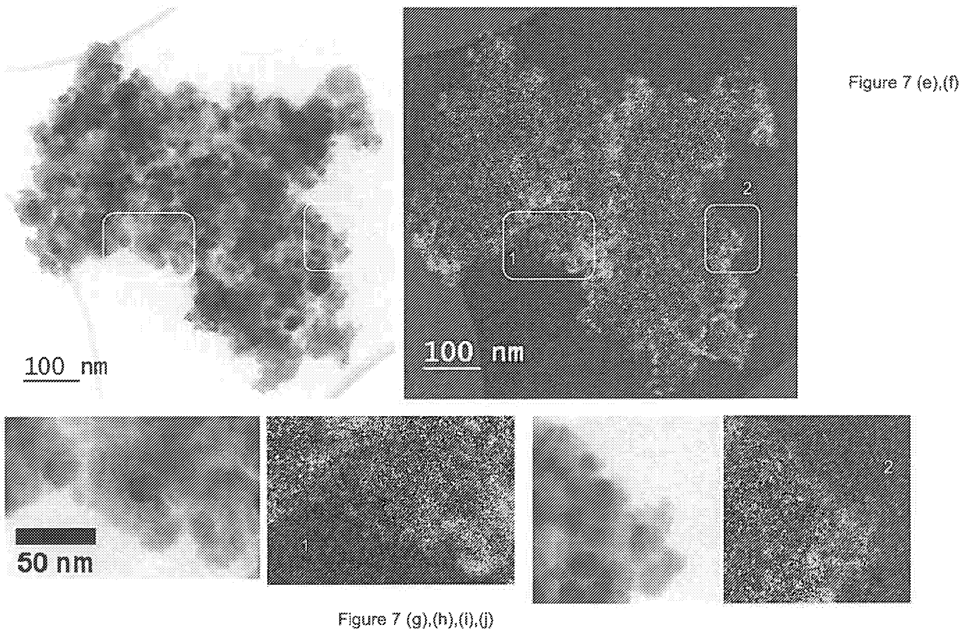

La figure 7 concerne un catalyseur 10%Co/10%TiO₂/SiC selon l'invention. Elle montre une micrographie obtenue par microscopie électronique à transmission (TEM) (figure 7(a)) et en mode filtrage de l'énergie (EFTEM) (figures 7(b), 7(c), 7(d) respectivement pour la cartographie du Si, du Ti et du Co)) d'une zone du catalyseur 10% Co / 10%TiO₂ / β-SiC selon l'exemple 7. La figure 7(d) permet d'estimer une taille moyenne des particules de cobalt à environ 20 nm, la longueur de la barre étant de 100 nm. Les figures 7(e) et 7(f) montrent les mêmes images que les figures 7(a) et 7(c) ; elles sont légèrement agrandies. Les zones marquées (1) et (2) sur la figures 7(e) et 7(f) sont agrandies sur les figures 7(g) et (h) pour la zone (1) et sur les figures 7(i) et (j) pour la zone (2), sachant que les figures 7(g) et (i) sont des micrographies TEM et les figures 7(h) et (j) des micrographies EFTEM du titane ; la longueur de la barre est de 50 nm.

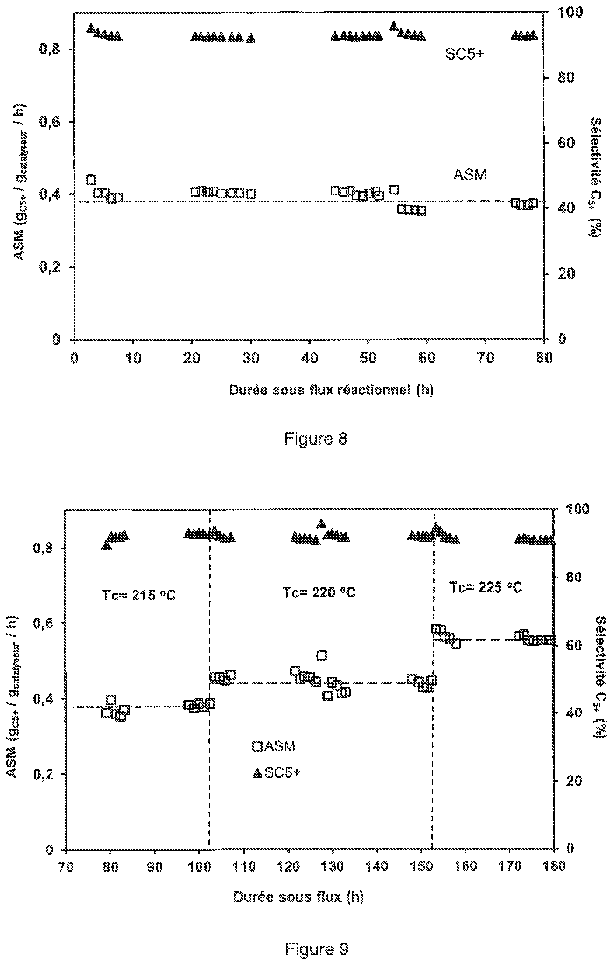

La figure 8 montre l'activité (ASM) et la sélectivité en hydrocarbures liquides (SC5+) obtenues avec le catalyseur 10%Co / 10%TiO₂/ β-SiC selon l'exemple 7 en fonction de la durée de l'essai. Le rapport H_{2 /} CO était de 2, la température de réaction était de 215°C, la pression totale 40 atmosphères, la vitesse volumique spatiale horaire (GHSV) 2850 h⁻¹.
La figure 9 montre l'activité (ASM) et la sélectivité en hydrocarbures liquides (SC5+) obtenues sur le catalyseur 10% Co / 10% TiO₂ / β-SiC selon l'exemple 7 en fonction de la température de réaction. Le rapport H₂ / CO était de 2, la pression totale 40 atmosphères, la vitesse volumique spatiale horaire (GHSV) = 3800 h⁻¹.

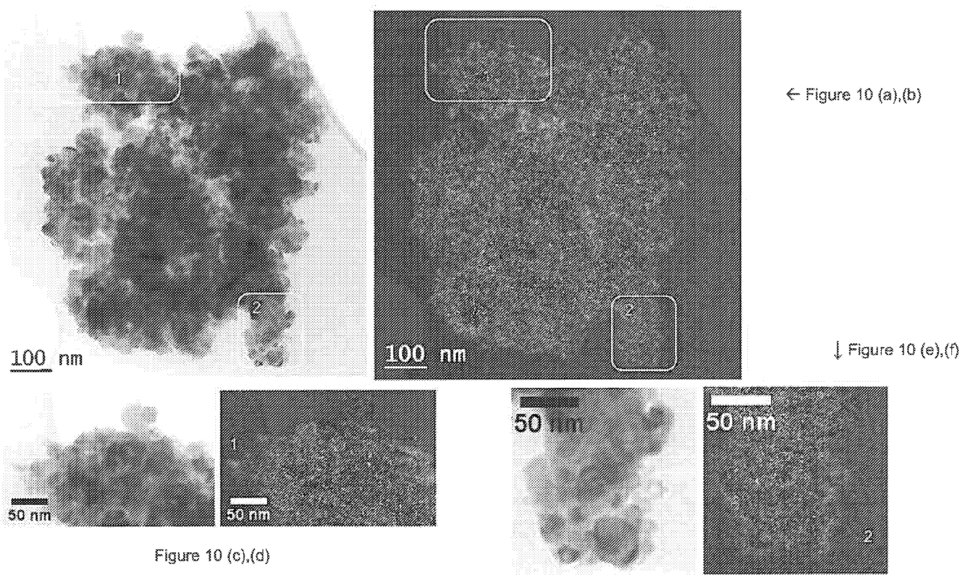

La figure 10 montre une deuxième zone du même échantillon que la figure 7. Les figures 10(a),(c) et (e) sont des micrographies TEM, les figures 10(b), (d) et (f) des micrographies EFTEM du titane. Les figures 10(c) et (d) correspondent à la zone (1), les figures 10(e) et (f) à la zone (2).

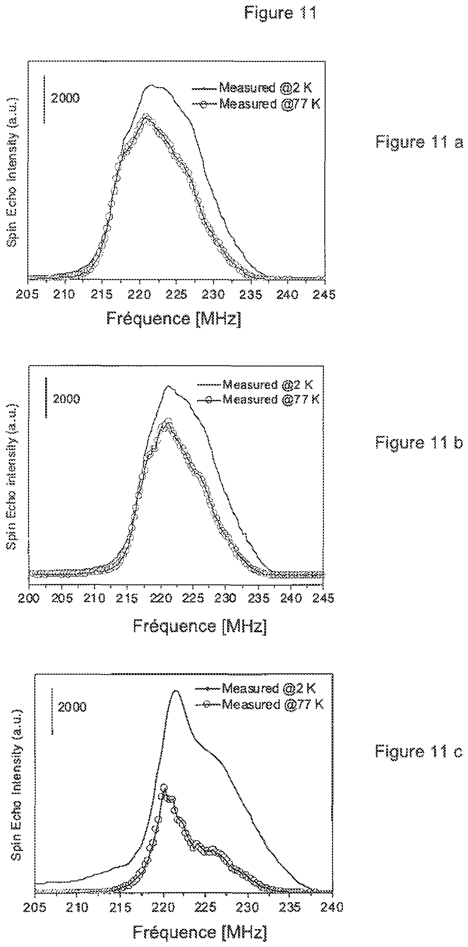

La figure 11 montre des spectres obtenus par résonance magnétique nucléaire du ⁵⁹Co sur des catalyseurs à base de cobalt déposés sur différents supports : 10%Co / SiC selon l'exemple n° 5 (comparatif)(figure 11a), 10%Co / 21%TiO₂ - 79%SiC selon l'exemple n° 9 (comparatif)(figure 11b), 10%Co / 10%TiO₂ / SiC selon l'exemple n° 7 (figure 11c). L'intensité de l'écho de spin est représentée en unités arbitraires. Les courbes pleines ont été enregistrées à une température de 2K, les courbes en points à 77K.

### Description détaillée

### 1. Définitions

Dans le cadre de la présente invention, le terme « surface spécifique » signifie la surface spécifique déterminée selon la méthode de Brunauer, Emmet et Teller (méthode BET), bien connue de l'homme du métier et décrite notamment dans la norme NF X 11-621.

La porosité d'un matériau est habituellement définie par référence à trois catégories de pores qui se distinguent par leur taille : la microporosité (diamètre inférieur à 2 nm), la mésoporosité (diamètre compris entre 2 nm et 50 nm) et la macroporosité (diamètre supérieur à environ 50 nm).

Dans certains modes de réalisation de la présente invention, on utilise une mousse de β-SiC qui se présente comme une mousse alvéolaire à porosité ouverte. Nous entendons ici par « mousse alvéolaire » une mousse qui présente à la fois une très faible densité et un grand volume poreux. La taille de l'ouverture des alvéoles est variable et se situe typiquement entre environ 800 et 6000 µm. Une telle mousse peut être préparée à l'aide de techniques connues. Elle présente une très faible microporosité. La mésoporosité est liée essentiellement aux ponts qui forment les alvéoles. La macroporosité ouverte d'une telle mousse peut varier de 30 à 95%, notamment de 50 à 90%, et sa densité volumique peut être comprise entre 0,05 g/cm³ et 0,5 g/cm³. D'une manière générale, pour son application comme support de catalyseur ou catalyseur, en dessous d'une densité de 0,05 g/cm³, on rencontre des problèmes de tenue mécanique de la mousse, alors qu'au-dessus de 0,5 g/cm³, le volume poreux alvéolaire va être réduit et la perte de charge va augmenter sans procurer un avantage fonctionnel. Avantageusement, la densité est comprise entre 0,1 et 0,4 g/cm³.

Dans d'autres modes de réalisation de la présente invention on utilise du β-SiC sous la forme d'extrudés, de granulés, de microbilles ou de grains. Ce matériau peut être préparé à l'aide de techniques connues.

### 2. Mode de synthèse du support

Selon un mode de réalisation, un support β-SiC de haute porosité est imprégné avec une solution organique d'un précurseur de TiO₂. Ledit précurseur de TiO₂ peut être un précurseur organique, notamment un alcoolate. On préfère le Ti(i-OC₃H₇)₄ (abrégé TIPP) Le solvant peut être un alcool, par exemple de l'éthanol ou du i-propanol. Avantageusement on utilise une solution d'éthanol (de préférence anhydride pour éviter l'hydrolyse du TIPP) contenant du Ti(i-OC₃H₇)₄ (distribué par exemple par la société Acros).

Dans un mode de réalisation avantageux, le rapport molaire Ti / Si est compris entre 2,5% et 10% (soit une charge en TiO₂ entre 5% et 20% massiques par rapport à la masse totale de SiC). Après imprégnation le solide est séché, par exemple dans une étuve à 110 °C pendant 8 heures. Avantageusement, on laisse le solide après l'imprégnation à la température ambiante (par exemple pendant 4 heures) avant de procéder au séchage. La transformation du précurseur en TiO₂ est effectuée par une calcination (avantageusement sous air), de préférence à une température comprise entre environ 400°C et environ 1000°C). La pente de montée en température est avantageusement comprise entre 1,5°C/min et 3,5°C/min, et de préférence d'environ 2°C/min. A titre d'exemple, la température de calcination peut être de 600 °C, et la durée du traitement à cette température peut être de 5 heures.

A titre de comparaison, on peut également préparer un matériau hybride contenant des teneurs en TiO₂ et en SiC similaires, mais dans lequel les deux phases se répartissent dans la totalité de la masse solide, par opposition aux matériaux préférés ici dans lesquels un cœur de SiC est recouvert par une couche de TiO₂ ; un tel support hybride n'entre pas dans le cadre de la présente invention.

Afin d'éviter toute confusion dans les notations, on convient de noter dans ce qui suit « x%TiO₂/SiC » les solides formés par une couche superficielle de TiO₂ (ou au moins par des particules individuelles de TiO₂) déposée sur un support SiC à raison d'une teneur à x % par rapport à la masse de SiC, et « y%TiO₂-SiC » les solides mixtes (appelés ici aussi : « hybrides ») contenant y% de TiO₂ et (100-y)% de SiC répartis dans la masse du matériau, sachant que x et y expriment des pourcentages massiques.

Le procédé de préparation du support assure une bonne dispersion des fines particules de TiO₂ sur toute la surface macro-poreuse et méso-poreuse, externe et interne, du support. Il évite la formation de couches épaisses ou de croûtes. Le dépôt de cristallites de TiO₂ à partir d'une dispersion liquide selon l'état de la technique (formant une couche que l'homme du métier appelle « wash coat ») ne permet pas d'obtenir une dispersion aussi fine en profondeur du support de β-SiC.

Le support de β-SiC peut avoir toute forme géométrique appropriée. Il peut s'agir notamment de granules extrudées. Il peut également s'agir de mousse de β-SiC. Ces supports sont connus en tant que tels et peuvent être préparés par l'une des méthodes connues, à savoir : (i) l'imprégnation d'une mousse polyuréthane avec une suspension d'une poudre de silicium dans une résine organique (procédé Prin, voir EP 0 624 560 B1,

EP 0 836 882 B1 et EP 1 007 207 A1) ; (ii) la réaction entre des vapeurs de SiO avec du carbone réactif à une température comprise entre 1100°C et 1400°C (procédé Ledoux, voir EP 0 313 480 B1) ; ou (iii) la réticulation, carbonisation et carburation d'un mélange d'un prépolymère liquide ou pâteux et d'une poudre de silicium (procédé Dubots, voir EP 0 440 569 B1 et EP 0 952 889 B1).

### 3. Mode de préparation du catalyseur sur le support

Sur le support TiO₂/SiC on dépose ensuite une phase active constituée d'un ou plusieurs métaux de transition. On préfère le fer, le cobalt ou un mélange des deux. Ce dépôt est réalisé avantageusement par la méthode d'imprégnation du volume poreux par une solution d'un précurseur de la phase active, qui est connue de l'homme du métier. Ledit précurseur peut être une solution d'au moins un composé organo-métallique du métal qui constituera la phase active, ou d'un sel organique de ce dernier. Après l'imprégnation, le solide est séché (de préférence à une température comprise entre 100°C et 140°C) puis calciné (de préférence sous air à une température comprise entre 250°C et 450°, et de préférence avec une pente de chauffe comprise entre 0,6°C/min et 1,6°C/min) pour obtenir un oxyde dudit métal. La phase active est obtenue par réduction du précurseur oxyde, de préférence à une température comprise entre 200°C et 380°C (et de préférence avec une pente de chauffe comprise entre 2°C/min et 4°C/min).

La taille moyenne (d) des particules de phase active (i.e. leur diamètre moyen) est avantageusement comprise entre 15 nm et 40 nm. Elle peut être estimée soit à partir de la taille du précurseur oxyde, qui est chimiquement stable et donc plus facile à manipuler en vue de sa caractérisation (pour le fer et le cobalt, la taille des particules est approximativement 0,75 fois celle des particules d'oxyde), soit à partir de l'élargissement du pic de diffraction selon la formule de Scherrer, bien connue : *d* = kλ/(τ. cos θ) où λ représente la longueur d'onde du rayonnement incident, τ la largeur à mi-hauteur du pic de diffraction, k est une constante et θ est la moitié de déviation de l'onde.

Dans un mode de réalisation avantageux de cette étape, après imprégnation le solide est séché à l'air ambiant pendant 4 heures puis dans une étuve à 110°C pendant 8 heures. Le solide ainsi séché est calciné sous air à 350°C (pente de 1°C/min) pendant 2 heures afin d'obtenir le précurseur oxyde du catalyseur, Co₃O₄/xTiO₂/SiC. Le catalyseur est obtenu par réduction du précurseur oxyde sous flux d'hydrogène à 300°C (pente de 3°C/min) pendant 6 heures. Le catalyseur est noté ensuite yCo/xTiO₂/SiC avec y qui représente la charge (en pourcent) de cobalt sur le catalyseur ([Co]/[TiO₂+SiC] et x qui représente la charge (en pourcent) du TiO₂ sur le support SiC ([TiO₂]/[SiC]). La taille moyenne des particules de Co est d'environ 20 nm.

### 4. Utilisation du catalyseur

Le catalyseur ainsi préparé est spécialement adapté pour la réaction Fischer-Tropsch, et plus généralement pour la conversion catalytique d'un mélange de CO et d'hydrogène en hydrocarbures. Le support peut avoir toute forme géométrique appropriée, et peut se présenter notamment sous la forme de granules, billes, microbilles, extrudés, ou encore sous la forme de plaques ou cylindres de mousse.

Les meilleurs résultats sont obtenus avec un catalyseur dont le support présente un rapport massique TiO₂ / SiC compris entre 8% et 16%, et de manière optimale entre 8% et 13%.

### 5. Avantages

Les avantages supplémentaires dans l'utilisation d'un tel système catalytique par rapport à ceux reportés à l'heure actuelle dans la littérature sont les suivants :
(i) une mise en forme facile du support en fonction de la nature du réacteur mis en jeu,
(ii) un parfait contrôle de la distribution méso- et macro-poreuse du support permettant une meilleure accessibilité des réactifs et une meilleure évacuation des produits intermédiaires de la réaction,
(iii) une conductivité thermique plus élevée du support, par rapport à la silice ou l'alumine, permettant de réduire la formation des points chauds sur la surface du catalyseur, sachant que les points chauds peuvent induire une dégradation de la sélectivité et aussi favoriser le frittage des particules de la phase active.
(iv) une tenue mécanique supérieure par rapport aux supports macroscopiques en TiO₂ (extrudés, mousse, anneaux, billes etc.) et une meilleure résistance à l'attrition, car le TiO₂ se trouve dans la porosité du support et non pas sur sa surface. Cette résistance à l'attrition est une propriété particulièrement importante si le catalyseur est utilisé sous la forme de microbilles dans un réacteur de type « slurry ».

### Exemples

### Exemples n°1 à 3 (selon l'invention): Dépôt de TiO₂ sur support SiC

On a approvisionné des grains de SiC poreux présentant une surface spécifique de 40 m²/g et une distribution poreuse exempte de micropores. Le volume poreux des grains a été imprégné avec une solution d'éthanol contenant du Ti(i-OC₃H₇)₄ en quantité nécessaire pour déposer une charge de Ti correspondant à 5% de TiO₂, 10% de TiO₂ et 15% de TiO₂ par rapport au poids de SiC, respectivement pour les matériaux des exemples n°1, 2 et 3.

Après imprégnation les solides ont été laissés à température ambiante pendant 4 heures puis séchés en étuve à 110 °C pendant 8 heures. La transformation du sel précurseur en TiO₂ a ensuite été effectuée par calcination sous air à 600 °C pendant 5 heures avec une pente de montée en température de 2 °C/min. Les matériaux ainsi obtenus présentent des surfaces spécifiques respectivement de 38 m²/g, 41 m²/g et 41 m²/g pour les exemples 1, 2 et 3. La distribution poreuse du SiC de départ et du support 10%TiO₂/SiC sont reportées sur la figure 1A.

La figure 4(b) montre une image EFTEM (Energy Filtered Transmission Electron Microscopy) obtenue sur le support 10%TiO₂/SiC. L'image n°4(a) à gauche est une image TEM en coupe permettant d'avoir une vue d'ensemble de l'échantillon. L'image de la figure 4(b) montre clairement la présence d'une fine couche de TiO₂ recouvrant la surface poreuse du grain de SiC.

### Exemple n°4 (comparatif): Préparation d'un support mixte TiO₂-SiC contenant 21% de TiO₂ et 79% de SiC

Un matériau mixte TiO₂-SiC a été préparé de la manière suivante :
On a approvisionné 1620 g de poudre de silicium, 1520 g de résine phénolique solide Novolaque, 600 g de poudre de TiO₂ (P25 de Degussa-Evonik, surface BET environ 50 m²/g, taille de particules moyenne de l'ordre de 20 nm), 78 g d'Hexamethylène-tetramine (HMT), 30 g de plastifiant en poudre Zusoplast PS1, 200 g d'une solution à 35% d'alcool polyvinylique et 1195 g d'eau.

On a mélangé les poudres. On a dilué l'alcool polyvinylique dans la quantité d'eau. On a préparé un mélange extrudable en introduisant sous agitation le mélange liquide sur les poudres. On a extrudé ce mélange pour former des granulés de 3 mm de diamètre. Après séchage à l'air ambiant puis à 150°C pendant 4h, les granulés ont été traités à 1360°C sous flux d'argon pendant une heure. Le solide obtenu comporte 83,7% massiques de SiC et 16,3% massique de TiC (soit une fraction molaire de 11,5% de Ti par rapport à la somme Ti + Si).

Le diagramme de diffraction des rayons X indiquait que le solide obtenu est un mélange de SiC et de TiC (« composite SiC-TiC »). Sa surface spécifique BET était de 54 m²/g, dont 27 m²/g de surface microporeuse.

Ensuite, ce composite SiC-TiC a été oxydé sous air à 400°C pendant 8h. On a obtenu alors un composite 20,6% massiques TiO₂ - 79,4% massiques SiC (soit une fraction molaire de 11,5 % de Ti par rapport à la somme Ti + Si) qui présente une résistance mécanique de 59 N/mm. Sa surface spécifique est de 83 m²/g, dont 53 m²/g de surface microporeuse. La figure 5 montre que la surface de ce matériau est constituée zones juxtaposées de SiC et de TiO₂ en quantités équivalentes.

Ce matériau présente donc une composition similaire à celle de l'exemple 2, mais ses propriétés poreuses d'une part et sa composition de surface d'autre part en font un support catalytique très différent.

### Exemple 5 (comparatif) : préparation d'un catalyseur à 10%Co/SiC

Un catalyseur à 10% de cobalt ne contenant pas de titane a été préparé à partir du SiC brut déjà utilisé dans les exemples 1 à 3.

On a préparé une solution aqueuse de nitrate de cobalt que l'on imprègne sur le SiC par la méthode du volume poreux. La concentration de nitrate de cobalt est calculée de manière à obtenir la charge souhaitée en cobalt dans le catalyseur final. Le solide a ensuite été séché à l'air ambiant pendant 4h, puis en étuve à 110°C pendant 8h. Il a subi ensuite une calcination sous air à 350°C (pente de 1°C/min) pendant 2 heures afin d'obtenir le précurseur oxyde du catalyseur, Co₃O₄/SiC. Le catalyseur 10%Co a été obtenu par réduction du précurseur oxyde sous flux d'hydrogène à 300°C (pente de 3°C/min) pendant 6 heures. Sa surface spécifique était de 33 m²/g. La taille moyenne des particules de Co est estimée à 40-50 nm (voir tableau 1).

### Exemples 6 à 8 (selon l'invention) : préparation de catalyseurs à 10%Co/TiO₂/SiC

L'exemple 5 a été reproduit en remplaçant le support SiC par les solides préparés selon les exemples 1 à 3. On a obtenu alors respectivement les catalyseurs selon les exemples 6, 7 et 8, contenant tous une charge de 10% de Co. La surface spécifique et le diamètre moyen des particules de Co mesurés sur ces catalyseurs sont reportés dans le tableau 1.

Par rapport aux surfaces spécifiques des supports initiaux, les catalyseurs à 10% de Co déposés sur les supports recouverts d'une couche de TiO₂ ne montrent pas de modification sensible de la surface spécifique, alors que celle-ci diminue de 40 m²/g à 33 m²/g après dépôt de Co sur SiC.

La présence du TiO₂ influe d'une manière significative la taille moyenne des particules de cobalt. En effet, celle-ci passe ainsi d'environ 40-50 nm sur SiC à 20 nm lorsque le support a été préalablement recouvert d'une couche à 10% en poids de TiO₂.

Les diagrammes de diffraction des rayons X des catalyseurs sont présentés sur la figure 2. Les pics de diffraction de la phase TiO₂ sont clairement visibles sur les diagrammes de diffraction. La diffraction indique aussi que la réduction est (relativement) complète car on n'observe pas de pics de diffraction correspondants à la phase oxyde de cobalt, CoO et/ou Co₃O₄. L'agrandissement du pic de diffraction du cobalt (insert de la figure 2) sur les diagrammes montre qu'il y a une augmentation de la largeur à mi-hauteur de ce pic indiquant que la taille des particules de cobalt participant à la diffraction cohérente est plus petite en présence du TiO₂.

Les images MEB des catalyseurs de l'exemple 5 et 7 (10%Co/SiC et 10%Co/10%TiO₂/SiC) sont présentées sur la figure 3 et indiquent une diminution importante de la taille des particules de cobalt en présence du TiO₂. Ces résultats sont en accords avec ceux obtenus par la diffraction des rayons X présentés précédemment : la dispersion des particules de cobalt est donc fortement améliorée par la présence de la couche superficielle de TiO₂.

Les images EFTEM (Energy Filtered Transmission Electron Microscopy) obtenues sur le catalyseur 10%Co/10%TiO₂/SiC sont présentées sur la figure 7. Les figures 7(c) et 7(d) montrent la dispersion des particules de cobalt sur la couche de TiO₂.On constate sur la cartographie de la figure 7(d) que la taille moyenne des particules de cobalt est relativement petite (de l'ordre de 20 nm) et homogène. Ce résultat est en bon accord avec ceux obtenu à partir de l'élargissement des pics de diffraction du cobalt présentés précédemment (tableau 1). La figure 10, qui se réfère à une autre zone du même échantillon, corrobore ces résultats et conclusions.

### Exemple 9 (comparatif) : préparation d'un catalyseur à 10%Co/21% TiO₂-SiC

Un catalyseur à 10% de Co a été préparé selon l'exemple 5 en remplaçant le support SiC par le solide mixte 21%TiO₂-SiC préparé dans l'exemple 4. Les figures 5 et 6 montrent que le catalyseur obtenu présente deux populations de phase active : de très grosses particules de Co localisées en surface des zones de SiC et quelques très petites particules de Co déposées sur des zones de TiO₂. Le solide préparé à l'exemple 4 présente une surface spécifique de 83 m²/g et une fraction importante de pores de diamètre inférieur à 10 nm. Après dépôt de 10% de Co, la surface spécifique chute à 25 m²/g. La figure 1C reporte les distributions poreuses de ce support, ainsi que celle du catalyseur obtenu après dépôt de 10% de Co. On observe que les pores de petit diamètre ont disparus après dépôt de la phase active.

### Exemple 10 (comparatif) : Préparation et essai d'un catalyseur à 10%Co/SiC

On a préparé un catalyseur comprenant une phase active de cobalt (déposée par le procédé décrit dans l'exemple 5) sur un support de SiC microporeux d'environ 58 m²/g, dont 27 m²/g de micropores. Après le dépôt de la phase active, la surface spécifique mesurée n'est plus que de 24 m²/g. La figure 1C reporte les distributions poreuses de l'échantillon SiC de départ, ainsi que celle du catalyseur après dépôt de 10% de Co. Les pores de faible diamètre présents sur le support d'origine ont disparu après dépôt de la phase active, et ne sont donc d'aucune utilité pour la réaction catalytique.

### Exemple 11 : Evaluation des performances catalytiques des différents catalyseurs selon l'invention ou selon les exemples comparatifs.

Les performances des catalyseurs préparés selon les exemples 5, 6, 7, 8 et 9 ont été évaluées dans la réaction Fischer-Tropsch. Les résultats sont reportés dans le tableau 2. On observe un doublement d'activité catalytique pour le catalyseur contenant une couche continue de 10% de TiO₂ sur le SiC par rapport au catalyseur préparé sur SiC seul. Les essais catalytiques indiquent que la concentration en TiO₂ a une influence significative sur les performances catalytiques des catalyseurs à base de cobalt avec une concentration massique en cobalt de 10%.

Les résultats à 215°C montrent que l'activité dans la réaction Fischer-Tropsch (exprimée en termes de CoTY) est optimale pour les catalyseurs chargés avec 10% massiques de cobalt avec une concentration massique en TiO₂ de 10%, tandis qu'elle est deux fois moins élevée pour les charges en TiO₂ plus faibles (5% massiques) ou plus élevées (15% massiques). Cependant, il est à noter que cette concentration optimale en agent dopant pourrait être différente en fonction de la charge réelle en cobalt. En effet, la valeur optimale de la charge en TiO₂ pourrait être amenée à être différente lorsque la charge en cobalt est augmentée, par exemple en passant de 10% à 30% massiques.

Il est à noter que la sélectivité en C₅₊ est légèrement plus faible sur le catalyseur avec 10% massiques de TiO₂ par rapport aux deux autres catalyseur. Ceci pourrait être dû à une conversion plus élevée dans le cas du catalyseur chargé avec 10% de TiO₂. La diminution de la sélectivité pourrait être due à une taille de particules de cobalt plus petite qui ont tendance à produire des produits hydrocarbures légers lors de la réaction Fischer-Tropsch.

Nous discutons ici en plus grand détail les performances des catalyseurs préparés selon l'exemple 7. La stabilité des performances catalytiques de ces catalyseurs (10%Co/10%TiO₂/SiC) a été évaluée et les résultats sont présentés sur la figure 8. On constate clairement sur cette figure que l'activité et la sélectivité en hydrocarbures liquides du catalyseur sont extrêmement stables en fonction du temps de test.

L'influence de la température de réaction sur l'activité du catalyseur de l'exemple 7 (10%Co/10%TiO₂/SiC) lors du test Fischer-Tropsch a également été évaluée et les résultats sont présentés sur la figure 9 ainsi que dans le tableau 2. Ce tableau rassemble aussi d'autres résultats d'essais catalytiques obtenus avec des catalyseurs préparés selon d'autres exemples. Il est à noter que lors des essais avec le catalyseur selon l'exemple 7, la vitesse spatiale volumique horaire a été augmentée de 2850 h⁻¹ à 3800 h⁻¹, afin d'éviter d'atteindre des conversions trop élevées qui pourraient induire des problèmes d'emballement thermique dans le lit catalytique. Ces emballements thermiques pourraient modifier les caractéristiques de la phase active par frittage des particules de la phase active. L'activité en SFT augmente d'une manière significative avec la température de réaction tandis que la sélectivité des hydrocarbures liquides demeure élevée et stable. L'activité spécifique massique (ASM) atteint environ 0,6 g_{C5+}/g_{catalyseur}/h avec une sélectivité en hydrocarbures liquides autour de 90% à 225°C. Il est à noter également que le catalyseur présente une stabilité relativement élevée et aucune désactivation n'a été observée à chaque palier de test.

A titre de comparaison, le catalyseur préparé selon l'exemple 9 en déposant du Co sur un matériau mixte 21%TiO₂-SiC, présente une activité spécifique très inférieure à celle mesurée sur des catalyseurs faisant l'objet de l'invention.

Ces résultats démontrent que les catalyseurs préparés selon l'invention présentent de très bonnes performances catalytiques pour la réaction FT, une excellente stabilité dans le temps.

### Exemple n° 12 : Caractérisation des catalyseurs par RMN

L'exemple ci-dessous concerne l'influence de la nature du support sur la dispersion des particules de cobalt. Pour l'exemple trois catalyseurs à base de SiC ont été testés: sur SiC pur (noté 10%Co/SiC selon l'exemple comparatif n°5), sur SiC dopé avec 21 % de TiO₂ (noté 10%Co/21%TiO₂-79%SiC, selon l'exemple comparatif n°9) et sur SiC recouvert par une couche de TiO₂ déposée post-synthèse (noté 10%Co/10%TiO₂/SiC, selon l'exemple n°7). L'analyse de la dispersion des particules de cobalt sur les différents catalyseurs est réalisée par RMN du ⁵⁹Co (P. Panissod, C. Meny, Appl. Magn. Reson. 19, 447, 2000).

L'analyse par RMN du ⁵⁹Co indique que le nombre des particules de cobalt de taille inférieure à 8 nm a été fortement augmenté sur le catalyseur 10%Co/10%TiO₂/SiC par rapport à ceux des deux autres catalyseurs. En effet, le nombre d'atomes de cobalt formant des particules de taille < 8 nm, déterminé par différence entre la courbe obtenue à deux températures de blocage : 2 K et celle à 77 K (voir Figure n°11), est d'environ 70% sur le catalyseur 10%Co/10%TiO₂/SiC alors qu'il n'est que de 33% pour le catalyseur 10%Co/21%TiO₂-79%SiC et de 28% pour le catalyseur 10%Co/SiC. Les résultats ainsi obtenus confirment que le dépôt d'une couche de TiO₂ sur la surface du support SiC a permis d'améliorer d'une manière sensible la dispersion des particules de cobalt ce qui explique la forte amélioration de l'activité catalytique pour la synthèse de Fischer-Tropsch. Il est à noter cependant que la distribution de taille obtenue par RMN pourrait être légèrement modifiée d'un catalyseur à un autre et aussi qu'elle ne tient pas compte de la formation des agrégats. En effet, la formation des agrégats observée par microscopie électronique à transmission pourrait amener à sous-estimer la taille moyenne des particules de cobalt dans le catalyseur.

**Tableau 1**

| Caractéristiques des supports et des catalyseurs utiliséspour la synthèseF-T | | | |
|---|---|---|---|
| | Surface spécifique [m²/g] | Taille des particules de Co (d(Co)) ^{(a)} [nm] | Taille des particules de Co (d(Co) ^{(b)} [nm] |
| Support | | | |
| SiC | 40 | - | - |
| 5%TiO₂/SiC (exemple 1) | 38 | - | - |
| 10%TiO₂/SiC (exemple 2) | 41 | - | - |
| 15%TiO₂/SiC (exemple 3) | 41 | - | - |
| 21%TiO₂-79%SiC (ex. 4) (*) | 83 | - | - |

| Catalyseur | | | |
|---|---|---|---|
| 10%Co/SiC (ex. 5) (*) | 32,7 | 42 ± 5 | 51 ± 5 |
| 10%Co/5%TiO₂/SiC (ex. 6) | 36,7 | 31 ± 5 | 30 ± 5 |
| 10%Co/10%TiO₂/SiC (ex. 7) | 40,4 | 24 ± 5 | 24 ± 5 |
| 10%Co/15%TiO₂/SiC (ex. 8) | 40,7 | 34 ± 5 | NM |
| 10%Co/21%TiO₂-79%SiC (ex. 9) (*) | 25,0 | NM | NM |
| ^{(a)} *d*(Co) = 0,75 x *d*(Co₃O₄) | ^{(b)}*d*(Co) = kλ/(τ. cos) déterminé à partir du plande diffraction (111) f.c.c. de particules de cobalt | | (*) ex. comparatif |

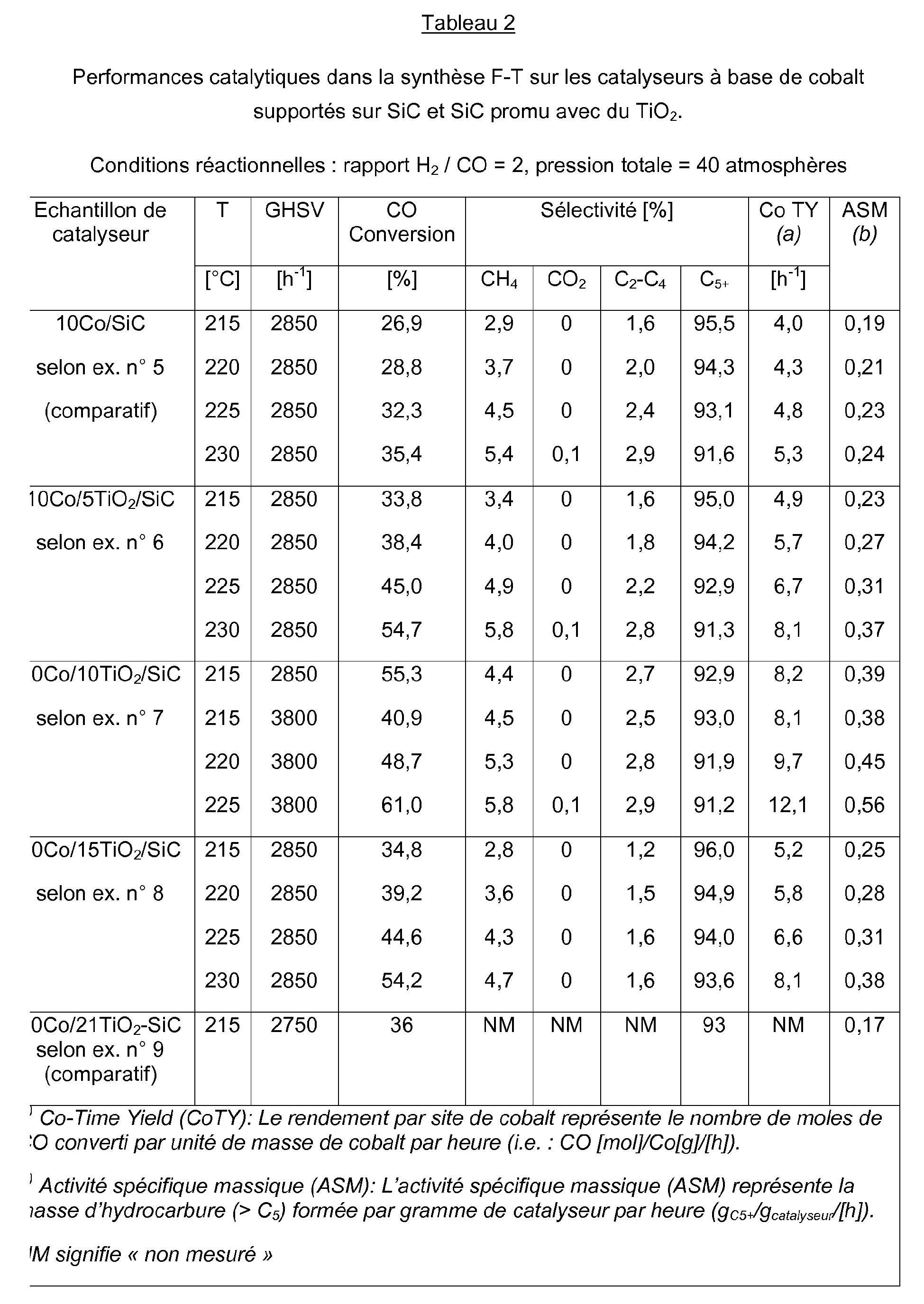

## Revendications

1. Utilisation, pour la réaction de Fischer-Tropsch, d'un catalyseur obtenu par le dépôt d'une phase active sur un support de catalyseur, ladite phase active étant du cobalt métallique, du fer métallique ou un mélange des deux, finement divisés, ledit support de catalyseur étant un support de catalyseur à base SiC recouvert au moins partiellement de TiO₂ susceptible d'être obtenu par un procédé de préparation comprenant les étapes suivantes :
(a) on approvisionne un support en β-SiC de haute porosité, de préférence sous la forme d'extrudés, granules, billes, microbilles, ou de mousse alvéolaire, et dont la surface spécifique est d'au moins 15 m²/g, et de préférence d'au moins 20 m²/g,
(b) on prépare une solution d'au moins un précurseur de TiO₂,
(c) on imprègne ledit support par ladite solution,
(d) on sèche ledit support imprégné,
(e) on calcine ledit support imprégné pour transformer ledit précurseur de TiO₂ en TiO₂, à une température comprise entre 400 °C et 1000 °C, et de préférence entre 500 °C et 900 °C.

2. Utilisation selon la revendication 1, **caractérisé en ce que** la contribution microporeuse à la surface spécifique dudit support en β-SiC est inférieure à 5 m²/g.

3. Utilisation selon l'une quelconque des revendications 1 ou 2, **caractérisée en ce que** dans ledit support de catalyseur le rapport massique TiO₂ / SiC est compris entre 3% et 30%, de préférence entre 5% et 20%, encore plus préférentiellement entre 8% et 16%, et de manière optimale entre 8% et 13%.

4. Utilisation selon la revendication 3, **caractérisée en ce que** dans ledit support de catalyseur la teneur en TiO₂ est inférieure à 0,02 g par m² de surface spécifique du support en SiC, et de préférence inférieure à 0,013 g par m² de surface spécifique du support en SiC, et encore plus préférentiellement inférieure à 0,009 g par m² de surface spécifique du support en SiC.

5. Utilisation selon l'une quelconque des revendications 1 à 4 d'un catalyseur, **caractérisée en ce que** ledit catalyseur comprend entre 5% et 40% massiques, et de préférence entre 10% et 25% massiques (exprimés par rapport à la masse du support) de cobalt sur un support dont le rapport massique TiO₂ / SiC est compris entre 5% et 20%.

## Patentansprüche

1. Verwendung eines Katalysators, der durch Abscheidung einer aktiven Phase auf einen Katalysatorträger erhalten wird, für eine Fischer-Tropsch-Reaktion, wobei die aktive Phase aus metallischem Kobalt, metallischem Eisen oder einem Gemisch von beiden, die fein verteilt sind, ist, wobei der Katalysatorträger ein zumindest zum Teil mit TiO₂ überzogener Katalysatorträger auf SiC-Basis ist, der durch ein Herstellungsverfahren erhalten werden kann, das die folgenden Schritte umfasst:
(a) Bereitstellen eines Trägers aus β-SiC mit hoher Porosität, vorzugsweise in der Form von Extrudat, Granulat, Kügelchen, Mikrokügelchen oder zelligem Schaum, und dessen spezifische Oberfläche mindestens 15 m²/g und vorzugsweise mindestens 20 m²/g beträgt,
(b) Herstellen einer Lösung von mindestens einem TiO₂-Vorläufer,
(c) Imprägnieren des Trägers mit der Lösung,
(d) Trocknen des imprägnierten Trägers,
(e) Kalzinieren des imprägnierten Trägers zum Umwandeln des TiO₂-Vorläufers zu TiO₂ bei einer Temperatur, die zwischen 400 °C und 1000 °C, vorzugsweise zwischen 500 °C und 900 °C liegt.

2. Verwendung nach Anspruch 1, **dadurch gekennzeichnet, dass** der Mikroporenbeitrag an der spezifischen Oberfläche des Trägers aus β-SiC geringer als 5 m²/g ist.

3. Verwendung nach einem der Ansprüche 1 oder 2, **dadurch gekennzeichnet, dass** das TiO₂/SiC-Massenverhältnis in dem Katalysatorträger zwischen 3 % und 30 %, vorzugsweise zwischen 5 % und 20 %, noch mehr bevorzugt zwischen 8 % und 16 % und optimal zwischen 8 % und 13 % liegt.

4. Verwendung nach Anspruch 3, **dadurch gekennzeichnet, dass** der TiO₂-Gehalt in dem Katalysatorträger niedriger als 0,02 g pro m² der spezifischen Oberfläche des Trägers aus SiC und vorzugsweise niedriger als 0,013 g pro m² der spezifischen Oberfläche des Trägers aus SiC und noch mehr bevorzugt niedriger als 0,009 g pro m² der spezifischen Oberfläche des Trägers aus SiC ist.

5. Verwendung nach einem der Ansprüche 1 bis 4 eines Katalysators, **dadurch gekennzeichnet, dass** der Katalysator zwischen 5 Massen-% und 40 Massen-% und vorzugsweise zwischen 10 Massen-% und 25 Massen-% (bezogen auf die Masse des Trägers ausgedrückt) Kobalt auf einem Träger umfasst, dessen TiO₂/SiC-Massenverhältnis zwischen 5 % und 20 % liegt.

## Claims

1. Use, for the Fischer-Tropsch reaction, of a catalyst obtained by the deposition of an active phase on a catalyst support, said active phase being metallic cobalt, metallic iron or a mixture of the two, finely divided, said catalyst support being an SiC-based catalyst support at least partially covered with TiO₂ capable of being obtained by a preparation method including the following steps:
(a) a highly porous β-SiC support is provided, preferably in the form of extrudates, pellets, beads, microbeads or cellular foam, and of which the specific surface is at least 15 m²/g, and preferably at least 20 m²/g,
(b) a solution of at least one TiO₂ precursor is prepared,
(c) said support is impregnated by said solution,
(d) said impregnated support is dried,
(e) said impregnated support is calcined in order to transform said TiO₂ precursor into TiO₂, at a temperature of between 400°C and 1000°C, and preferably between 500°C and 900°C.

2. Use according to claim 1, **characterized in that** the microporous contribution to the specific surface of said β-SiC support is less than 5 m²/g.

3. Use according to any one of claims 1 or 2, **characterized in that**, in said catalyst support, the TiO₂/SiC mass ratio is between 3% and 30%, preferably between 5% and 20%, even more preferably between 8% and 16% and optimally between 8% and 13%.

4. Use according to claim 3, **characterized in that**, in said catalyst support, the TiO₂ content is less than 0.02 g per m² of specific surface of the SiC support, and preferably less than 0.013 g per m² of specific surface of the SiC support, and even more preferably less than 0.009 g per m² of specific surface of the SiC support.

5. Use according to any one of claims 1 to 4 of a catalyst, **characterized in that** said catalyst includes between 5% and 40% by mass, and preferably between 10% and 25% by mass (expressed with respect to the mass of the support) of cobalt on a support of which the TiO₂/SiC mass ratio is between 5% and 20%.
